# FASCICULE DE BREVET EUROPEEN

(11) **EP 0 719 332 B1**
(45) Date de publication et mention de la délivrance du brevet: **28.07.2004**
(21) Numéro de dépôt: 94925523.6
(22) Date de dépôt: 22.08.1994
(51) Int. Cl.: C12N 15/23, C12N 15/28, C12N 5/10, A61K 48/00, C07K 14/525, C07K 14/57

(54) **CELLULES RECOMBINANTES DE LA LIGNEE MONOCYTE-MACROPHAGE POUR LA THERAPIE GENIQUE**
REKOMBINANTE ZELLEN DER MONOZYTEN-MAKROPHAGEN ZELLLINIE ZUR GEN-THERAPIE
MONOCYTE MACROPHAGE LINE RECOMBINANT CELLS FOR GENE THERAPY

(30) Priorité: 25.08.1993 FR 9310222
(43) Date de publication de la demande: 03.07.1996
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: HADDADA, Hedi, F-94140 Alfortville (FR); LOPEZ, Manuel, F-94500 Champigny-sur-Marne (FR); PERRICAUDET, Michel, F-28320 Ecrosnes (FR)
(74) Mandataire: Becker, Philippe
(86) Numéro de dépôt international: PCT/FR1994/001019
(87) Numéro de publication internationale: WO 1995/006120

(56) Documents cités:
- WO-A-89/05349
- WO-A-92/11355
- WO-A-93/09815
- WO-A-93/10219
- WO-A-93/11230
- DE-A- 4 219 626
- BIOCHEMICAL AND BIOPHYSICAL RESEARCH COMMUNICATIONS., vol.195, no.3, 30 Septembre 1993, DULUTH, MINNESOTA US pages 1174 - 1183 HADDADA, H. ET AL. 'Efficient adeno-mediated gene transfer into human blood monocyte-derived macrophages'
- HUMAN GENE TRANSFER, vol.219, 11 Avril 1991 pages 51 - 61 STRATFORD-PERRICAUDET, L. & PERRICAUDET, M. 'Gene tranfer into animals : the promise of adenovirus'
- PROCEEDINGS OF THE NATIONAL ACADEMY OF SCIENCES OF USA., vol.86, no.17, Septembre 1989, WASHINGTON US pages 6748 - 6752 BORDIGNON, C. ET AL. 'Retroviral vector-mediated high-efficiency expression of adenosine deaminase (ADA) in hematopoietic long-term cultures of ADA-deficient marrow cells'

## Description

La présente invention conceme des compositions cellulaires, leur préparation, les compositions pharmaceutiques les contenant, et leur utilisation en thérapie. Plus particulièrement, elle concerne la culture et l'activation de cellules du système phagocytaire mononucléaire, et leur utilisation en thérapie cellulaire, par exemple en immunothérapie adoptive.

Les cellules du système phagocytaire mononucléaire comprennent les monocytes du sang périphérique, leurs précurseurs de la moelle osseuse ou du sang, et les macrophages tissulaires. Les monocytes sont formés dans la moelle osseuse qu'ils quittent après maturation en passant du sang périphérique vers les tissus. Les monocytes humains circulant dans le sang ont une demi-vie d'environ 3 jours. Lorsqu'il atteint les tissus, le monocyte est appelé macrophage. Le nombre total des macrophages tissulaires dépasse largement le nombre des monocytes circulant, par un facteur d'environ 400. Des macrophages sont trouvés partout dans le corps mais sont particulièrement nombreux dans le foie (cellules de Kupffer), dans les ganglions lymphatiques, dans les poumons, dans le péritoine, et dans la peau (cellules de Langerhans). La demi-vie exacte des macrophages tissulaires n'est pas connue mais semble se compter en mois plutôt qu'en jours. Enfin le passage des monocytes de la circulation générale aux tissus est irréversible.

Les monocytes et les macrophages sont connus pour avoir des fonctions nombreuses et importantes, incluant l'induction de réponses immunitaires en phases aiguës (Anonymous, Lancet ii (1985) 536-537), la régulation de l'hématopoïese (Sieff C.A. J. Clin. Invest. 79 (1987) 1549-1557), l'activation du système immun (Unanue E.R. Annu. Rev. Immunol. 2 (1984) 395-428) et de la coagulation (Prydz H., Allison A.C. Thromb. Haemost. 39 (1978) 582-591), la destruction des organismes et des cellules tumorales (Sharma S.D., Remington J.S. Lymphokines 3 (1981) 181-212 et Carswell E.A. et al., Proc. Natl. Acad. Sci. USA 72 (1975) 3666-3670) et la réparation tissulaire et la cicatrisation (Korn J.H. et al., J. Clin. Invest. 65 (1980) 543-54).

Dans tout ce qui suit, une lignée de monocytes-macrophages est définie comme une lignée du système mononucléaire comprenant les monocytes du sang périphérique, leurs précurseurs de la moelle osseuse ou du sang, et les macrophages tissulaires. Elle comprend également les monocytes obtenus par culture des cellules précurseur ainsi que les macrophages obtenus après culture de cellules monocytaires, dans les conditions détaillées dans les exemples (voir aussi Andressen R.et al., Cancer Res. 50:7450; Bartholeyns J et al., Anticancer Res. 11 (1991) 1201-1204; Lopez M. et al., Journal of Immunological Methods 159 (1993) 29-38). Les précurseurs des monocytes macrophages comprennent notamment les cellules souches pluripotentes, les cellules souches myéloïdes (CFU-GEMM), les cellules souches myélomonocytiques (CFU-GM), les CFU-M, les monoblastes et les promonocytes.

Actuellement, les monocytes-macrophages sont utilisés en immunothérapie adoptive pour le traitement de certains types de cancers chez l'homme. Ces cellules sont purifiées à partir du sang circulant des patients, cultivées ex vivo et activées par l'interféron γ pour induire leur différentiation et augmenter leur pouvoir tumoricide, puis réinjectées aux patients. Cependant, ce traitement est assez lourd pour le malade car les monocytes-macrophages doivent être prélevés de façon régulière et fréquente, l'activation ex vivo nécessite des délais importants, et l'interféron γ est encore très cher. Pour cette raison, il est important de disposer de traitements plus efficaces, moins astreignants pour le malade et moins coûteux. La présente invention apporte une solution avantageuse à ce problème. La demanderesse a en effet montré qu'il est possible, à l'aide de vecteurs appropriés, de transférer des gènes ex vivo dans les cellules monocytes-macrophages permettant ainsi de leur conférer des propriétés supérieures, tant de cytotoxicité que de stimulation du système immunitaire.

Un premier objet de l'invention réside donc dans une composition cellulaire comprenant des cellules de la lignée monocyte-macrophage différenciées contenant un acide nucléique recombinant porté par un vecteur adénoviral comprenant, sous le contrôle d'éléments de régulation permettant son expression, un ou plusieurs gènes thérapeutiques.

La présente invention permet ainsi d'obtenir de manière simple et efficace des monocytes-macrophages sains et actifs utilisables en immunothérapie adoptive pour le traitement de certaines pathologies, telles que les cancers. La présente invention permet également de conférer aux monocytes-macrophages des propriétés thérapeutiques nouvelles ou accrues par rapport aux monocytes-macrophages de l'organisme, notamment dans le domaine de la défense contre des agents infectieux, contre les cellules tumorales ou dans l'activation du système immunitaire. De telles cellules sont avantageusement utilisables pour le traitement curatif ou préventif de maladies infectieuses (notamment virales), autoimmunes, des déficiences immunitaires, ou encore dans un but vaccinal.

Au sens de l'invention, le terme gène thérapeutique désigne tout gène dont la transcription et éventuellement la traduction dans la cellule génère un produit ayant un effet thérapeutique. Il peut s'agir en particulier de tout ou partie d'une protéine thérapeutique (interleukine, interféron, facteur de nécrose des tumeurs, facteurs de stimulation des colonies, etc), d'un peptide antigénique pour la réalisation d'un vaccin ou la stimulation du système immunitaire, ou également d'un ARN antisens capable de réguler l'expression d'une protéine spécifique, telle que par exemple une protéine d'origine virale, ou également d'interférer avec le cycle d'infection et/ou de réplication d'un virus.

Avantageusement, le gène code pour tout ou partie d'une protéine capable de conférer aux cellules de ladite lignée des propriétés anti-infectieuses, anti-cancéreuses ou immunostimulantes nouvelles ou accrues.

Plus préférentiellement, le gène est choisi parmi les gènes codant pour les interférons (préférentiellement gamma), les facteurs de nécrose des tumeurs (préférentiellement alpha), les interleukines (IL1-12), les facteurs de stimulation des colonies (G-CSF, M-CSF, GM-CSF, etc), le gène MDR (multi drug resistance), ou un gène codant pour un antigène d'une particule infectieuse ou spécifique d'une tumeur (par exemple une protéine de surface d'un virus : protéine gp160 du virus HIV notamment, ou antigène Muc-1 caractéristique des tumeurs épithéliales).

Dans un mode particulier, la présente invention a pour objet des compositions cellulaires telles que définies ci-dessus dans lesquelles l'acide nucléique recombinant est porté par un vecteur adénoviral. L'utilisation d'un vecteur selon l'invention permet en particulier d'améliorer l'administration de l'acide nucléique dans les cellules, et également d'augmenter sa stabilité dans lesdites cellules, ce qui permet d'obtenir un effet durable. De plus, il est possible d'introduire plusieurs gènes dans un même vecteur, ce qui augmente également l'efficacité du traitement.

Avantageusement, l'adénovirus utilisé est un adénovirus défectif. Le terme "adénovirus défectif' désigne un adénovirus incapable de se répliquer dans la cellule cible. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par l'acide nucléique recombinant. Préférentiellement, le virus défectif conserve néanmoins les séquences de son génome qui sont nécessaires à l'encapsidation des particules virales.

La demanderesse a en effet montré que les adénovirus étaient capables d'infecter de manière très efficace les cellules de la lignée monocyte-macrophage, de s'y maintenir stablement, et d'exprimer un gène thérapeutique, qu'il s'agisse d'un produit intracellulaire, d'un produit membranaire ou sécrété.

Il existe différents sérotypes d'adénovirus, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Les adénovirus recombinants défectifs utilisés dans le cadre de la présente invention peuvent être préparés par recombinaison homologue entre un adénovirus défectif et un plasmide portant entre autre la séquence d'acides nucléiques telle que définie ci-avant (Levrero et al., Gene 101 (1991) 195; Graham, EMBO J. 3(12) (1984) 2917). La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus recombinants défectifs, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12%).

Ensuite, les adénovirus qui se sont multipliés sont récupérés et purifiés selon les techniques classiques de biologie moléculaire.

Des techniques de construction de vecteurs dérivés des adénovirus, du HSV, du CMV ou des AAV, incorporant des séquences d'acides nucléiques hétérologues ont été décrites dans la littérature et peuvent être utilisées dans le cadre de la présente invention [Akli et al., Nature Genetics 3 (1993) 224; Stratford-Perricaudet et al., Human Gene Therapy 1 (1990) 241; EP 185 573, Levrero et al., Gene 101 (1991) 195 ; Le Gal la Salle et al., Science 259 (1993) 988 ; Roemer et Friedmann, Eur. J. Biochem. 208 (1992) 211 ; Dobson et al., Neuron 5 (1990) 353 ; Chiocca et al., New Biol. 2 (1990) 739 ; Miyanohara et al., New Biol. 4 (1992) 238 ; WO91/18088, WO90/09441, W088/10311].

L'infection des cellules de la lignée monocyte-macrophage différenciées peut être réalisée selon différents protocoles, à une multiplicité d'infection adaptable en fonction du vecteur utilisé, du gène concerné, etc, comme décrit dans les exemples ci-après.

Un mode particulier de réalisation de l'invention réside dans une composition cellulaire comprenant des cellules précurseurs de monocytes-macrophages contenant un adénovirus recombinant défectif comprenant, sous le contrôle d'éléments de régulation permettant son expression, un ou plusieurs gènes thérapeutiques. Plus particulièrement, les cellules précurseur sont choisies parmi les cellules souches et progéniteur du système hématopoïétique, et comprennent notamment les cellules souches pluripotentes, les cellules souches myéloïdes (CFU-GEMM), les cellules souches myélomonocytiques (CFU-GM), les CFU-M, les monoblastes et les promonocytes.

Comme indiqué plus haut, le gène thérapeutique est placé sous le contrôle d'éléments de régulation permettant son expression. Ces éléments de régulation sont généralement constitués par des séquences promotrices de la transcription. Il peut s'agir des séquences qui sont naturellement responsables de l'expression du gène thérapeutique considéré, lorsque ces séquences sont susceptibles de fonctionner dans les monocytes macrophages. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé comme vecteur. A cet égard, on peut citer par exemple les promoteurs des gènes E1A, MLP, CMV, RSV, etc. En outre, ces séquences d'expression peuvent être modifiées par addition de séquences d'activation, de régulation, etc. Ainsi, dans certains cas (par exemple lorsque le gène thérapeutique est ce lui de l'INF-γ ou du TNF-α), il est particulièrement avantageux d'utiliser comme éléments de régulation des promoteurs constitutifs, permettant une expression continue et élevée du gène therapeutique. Dans d'autres cas, il pourra être plus avantageux d'utiliser un promoteur régulé, dont l'activité pourra être controlée. Par ailleurs, lorsque le gène inséré ne comporte pas de séquences d'expression, il peut être inséré dans le génome du virus défectif en aval d'une telle séquence.

Les compositions cellulaires selon l'invention comprennent généralement une population de cellules enrichie en monocytes, macrophages ou leurs précurseurs. Il peut ainsi s'agir de monocytes, de macrophages, de leurs précurseurs, ou d'un mélange de ces différents types cellulaires. De préférence, les compositions selon l'invention possèdent plus de 80 % de monocytes, macrophages ou leurs précurseurs, et, encore plus préférentiellement, plus de 90 %.

Les monocytes-macrophages transformés ex vivo selon l'invention, par l'utilisation d'un vecteur adénoviral recombinant, se présentent donc comme un outil de choix pour la préparation d'une composition pharmaceutique, notamment anti-tumorale, anti-infectieuse, ou destinée à renforcer le système hématopoiétique d'un patient, notament son système immunitaire.

La présente invention a également pour objet une composition pharmaceutique contenant, à titre de principe actif, des cellules de la lignée monocytes-macrophages décrites ci-dessus.

Les compositions pharmaceutiques selon l'invention peuvent être administrées par voie systémique, par injection intratumorale, etc, en fonction des applications recherchées. Par ailleurs, elles peuvent être utilisées seules ou associées à d'autres compositions pharmaceutiques.

Dans ce qui précède, l'expression "associées" n'implique pas que les monocytes-macrophages et les autres médicaments soient nécessairement administrés en mélange ou de façon simultanée. Elle s'étend également à toute utilisation ou présentation impliquant leurs administrations à des intervalles de temps non nuls, mais suffisamment courts pour autoriser une addition ou une synergie dans les effets produits.

Dans un mode particulier de mise en oeuvre, la présente invention porte sur une composition pharmaceutique comprenant des cellules de la lignée monocyte-macrophage contenant un acide nucléique recombinant codant pour tout ou partie d'une protéine confèrant au macrophage la propriété d'éliminer tout ou partie de cellules tumorales d'un organisme.

Comme indiqué plus haut, les phagocytes mononucléaires ou monocytes nécessitent d'être différenciés en macrophages, puis activés, essentiellement par l'interféron gamma (INF γ), pour devenir des macrophages activés présentant une cytotoxicité vis-à-vis des cellules tumorales. Jusqu'à présent, la maturation du monocyte en macrophage et l'activation par l'interféron gamma étaient réalisées in vitro, à partir de cellules monocytaires prélevées à patir de cytaphérèses humaines, puis réinjectées après l'activation. Cependant les essais expérimentaux n'ont pas donné de résultats satisfaisants, en raison de problèmes d'efficacité de l'activation, du nombre élevé d'injections nécessaires (au moins une injection par semaine et donc une cytaphérèse par semaine), ce qui s'avère très lourd pour le patient.

La présente invention permet de générer ex vivo, à partir des monocytes-macrophages circulants ou de leurs précurseurs, des populations cellulaires enrichies en macrophages antitumoraux par transformation avec un ADN recombinant exprimant par exemple l'INF-γ.

Un mode préféré de la présente invention réside donc dans une composition pharmaceutique comprenant, à titre de principe actif, des cellules monocytes-macrophages telles que définies précédemment contenant un adénovirus recombinant défectif portant le gène de l'interféron γ. De telles cellules possèdent en effet des propriétés anti-cancéreuses accrues en immunothérapie adoptive, en raison d'une stimulation constante et pérenne des macrophages par l'interféron γ. L'invention permet ainsi la préparation de populations homogènes de macrophages humains tumorocides MAK (macrophages activated killers).

Parmi les avantages de cette composition selon l'invention par rapport aux traitements antérieurs, on peut citer sa reproductibilité, l'économie du traitement et le confort apporté au malade, lui évitant des cytaphérèses régulières.

Par ailleurs, parmi les médiateurs de l'activité antitumorale des macrophages, le TNF α (Tumor Necrosis Factor) joue également un rôle déterminant (Feinman et al., J. Immunol. 138 (1987) 635-640). Le TNF α (ou cachectine) est une cytokine relarguée essentiellement à partir de macrophages en réponse à une destruction tissulaire, une endotoxine bactérienne, une infection virale ou à d'autres cytokines (Quantin B. et al, Human Gene Transfer 219 (1991) 271-272). Il est également possible, dans le cadre de la présente invention, de préparer des macrophages ex vivo contenant un gène recombinant exprimant un tel médiateur, pour générer des population cellulaires très actives.

Un autre mode préféré de la présente invention réside donc dans une composition pharmaceutique comprenant, à titre de principe actif, des cellules monocytes-macrophages telles que définies précédemment contenant un adénovirus recombinant défectif portant un gène codant pour le TNF-α. Encore plus préférentiellement, les cellules de l'invention comportent un gène codant pour une forme membranaire du TNF-α. Cette expression membranaire de TNF α confère aux cellules transformées des propriétés anti-cancéreuses accrues lorsqu'elles sont utilisées en immunothérapie adoptive. Ce mode de réalisation permet en outre d'éviter un relargage du TNF dans le milieu extracellulaire, un tel relargage étant susceptible d'entraîner des effets de type inflammatoires extrêmement préjudiciables.

Avantageusement, l'acide nucléique recombinant code pour un TNF α deleté des acides aminés de la région N-terminale, ladite deletion portant au minimum sur 3 acides aminés et au plus sur 20 acides aminés, et de préférence sur les 12 acides aminés N-terminaux.

Dans un autre mode de mise en oeuvre, la présente invention porte sur une composition pharmaceutique comprenant des cellules de la lignée monocyte-macrophage contenant un acide nucléique recombinant codant pour tout ou partie d'une protéine confèrant au macrophage la propriété de prévenir ou de traiter les manifestations d'une infection par un agent infectieux tel un virus, un retrovirus, un parasite ou une bactérie, par induction ou régulation de la réponse immunitaire.

Cette utilisation met à profit la propriété des macrophages de présenter les antigènes à leur surface laquelle présentation antigènique stimule la réponse immunitaire des cellules immunocompétentes notamment les lymphocytes B et les lymphocytes T. Les antigènes présents dans l'organisme sont en effet captés par les macrophages, qui les traitent, puis les expriment à leur surface, en association avec les molécules du complexe d'histocompatibilité de classe II. La présente invention permet de développer de manière avantageuse cette propriété des macrophages, pour une stimulation générale ou spécifique du système immunitaire.

Pour la stimulation générale du système immunitaire, le gène thérapeutique peut être un gène recombinant exprimant un composé capable de stimuler la production des molécules MHC de classe II, permettant de générer des cellules selon l'invention ayant des propriétés de présentation antigénique accrues; et/ou un gène recombinant exprimant un antigène de surface viral ou bactérien, permettant de générer des cellules selon l'invention induisant une défense immunitaire renforcée contre ce type d'infection. A titre d'exemple de tels antigènes, on peut citer notamment la glycoprotéine du virus de la rage.

Pour une stimulation spécifique du système immunitaire, les macrophages peuvent être utilisés pour la présentation d'antigènes définis, dans un but vaccinal.

L'acide nucléique recombinant est porté de préférence par un vecteur adénoviral défectif dans les termes définis plus haut.

La présente invention a également pour objet un procédé de préparation de monocytes-macrophages tels que définis ci-avant, comprenant les étapes suivantes :
1. la culture des cellules de monocytes-macrophages ou de leurs précurseurs prélevés et isolés à partir de sang ou de moelle osseuse,
2. la transformation de ces cellules par un acide nucléique recombinant tel que défini ci-avant, et éventuellement,
3. le conditionnement et/ou le stockage des cellules ainsi obtenues.

Le prélèvement et l'isolement des cellules monocytes-macrophages ou de leurs précurseurs peuvent être effectués par toute technique connue de l'homme du métier. Ces différentes techniques peuvent impliquer des étapes physiques de séparation (centrifugation, tri cellulaire (FACS), etc), la sélection par des composés immunologiques (anticorps spécifiques de marqueurs cellulaires : US 4,965,204; EP 17 381, EP 30 450, etc) ou biochimiques (ligands de récepteurs membranaires : EP 405 972), etc.

Concernant les monocytes macrophages, ceux-ci peuvent être obtenus à partir du sang par différentes techniques, et notamment par cytaphérèse (Lopez et al., J. Immunol. Methods. 159 (1992) 29) ou par utilisation d'anticorps dirigés contre des marqueurs spécifiques des cellules monocytes macrophages tels que CD14, CD64 ou Max1. Ils peuvent également être obtenus ex vivo à partir de cellules précurseur, par culture dans des conditions appropriées permettant leur différenciation.

Concernant les cellules précurseur, celles-ci peuvent également être isolées au moyen d'anticorps reconnaissant des marqueurs spécifiques tels que : Cellules souches : CD33, CD34; Cellules CFU-GM et CFU-M : CD13, CD14, CD15, CD33, HLA DR; Monoblastes et promonocytes : CD13, CD14, CD15, CD33. Des techniques utilisables dans le cadre de la présente invention sont connues de l'homme du métier : voir par exemple les demandes EP451 611, W093/02182.

Les cellules ainsi obtenues peuvent ensuite être cultivées dans toute condition stérile permettant leur convervation, leur prolifération et/ou leur différentiation (s'il s'agit de cellules précurseur). La culture peut donc être effectuée simplement pour maintenir les cellules pendant la transformation. Il est en effet possible de transformer les cellules au stade de précurseur ou de monocytes, qui sont ensuite différenciés et/ou activés in vivo, après leur réadministration au patient. De même, la différencitation / activation peut être effectuée ex vivo, après la transformation. La culture peut également être effectuée pour permettre une prolifération et/ou une différentiation cellulaires, avant d'effectuer la transformation sur les cellules matures (monocytes, macrophages, MAK). En particulier, la prolifération des précurseurs et leur différentiation en cellules monocytaires peuvent être effectuées ex vivo en présence de facteurs de croissance et de différentiation, tels que le GM-CSF, le M-CSF, les interleukines IL3, IL4, IL6 et IL11, ou encore le SCF.

La culture des cellules isolées peut être effectuée dans différents milieux connus de l'homme du métier (exemple : RPMI, IMDM), supplémentés entre autres de sérum et d'acides aminés. La culture est réalisée en conditions stériles, de préférence à 37°C, comme illustré dans les exemples. Elle peut être efffectuée dans des plaques de culture ou, de préférence, dans des sacs en téflon.

Les conditions de culture peuvent être adaptées par l'homme du métier en fonction de la population cellulaire isolée, de l'utilisation recherchée, etc.

De même, la transformation des cellules par l'acide nucléique recombinant est effectuée en milieu stérile, dans des conditions adaptées par l'homme du métier en fonction de l'acide nucléique, de la population cellulaire isolée, etc.

Lorsque l'acide nucléique est porté par un adénovirus, l'infection des cellules de la lignée monocyte-macrophage peut être réalisée selon différents protocoles, à une multiplicité d'infection adaptable en fonction du vecteur utilisé et du gène concerné, comme décrit dans les exemples ci-après. Préférentiellement, les monocytes-macrophages sont incubés en présence de 50 à 250 pfu par cellule de virus purifié et, plus préférentiellement, de 80 à 100 pfu / cellule. Selon les conditions de la transformation, le pourcentage de cellules modifiées par insertion de l'acide nucléique recombinant peut varier de 30 à 95%.

Les cellules modifiées ainsi obtenues peuvent ensuite être conditionnées en vue d'une utilisation immédiate, et/ou stockées en vue d'une utilisation ultérieure.

Pour une réadministration immédiate, les cellules sont généralement mises en suspension dans un tampon phosphate ou dans du sérum physiologique, à une concentration variant de 10⁵ à 10⁹ cellules par dose.

Pour leur conservation, les cellules peuvent être congelées, de préférence en présence d'agents conservateurs tels que le glycérol, le DMSO, etc.

Les compositions cellulaires selon l'invention permet ainsi la réalisation d'une méthode de traitement comprenant l'administration de cellules telles que définies ci-avant. Il peut s'agir de cellules provenant du patient (autologues) ou d'un donneur (allogéniques).

L'administration peut être effectuée de différentes façons. Préférentiellement, les cellules sont administrées par injection, de préférence intraveineuse ou intratumorale. En outre, l'injection systémique peut être réalisée par perfusion.

Une méthode de traitement particulière comprend :
1. le prélèvement et l'isolement des monocytes-macrophages ou de leurs précurseurs à partir de sang ou de moelle osseuse,
2. la culture de ces cellules,
3. la transformation de ces cellules par un acide nucléique recombinant tel que défini ci-avant, éventuellement,
4. le conditionnement et/ou le stockage des cellules ainsi obtenues, puis,
5. leur administration au patient.

Le traitement de la présente invention offre de nombreux avantages par rapport aux autres traitements en immunothérapie adoptive par les LAK, les TIL, ou les NK (natural killers), tels que par exemple l'absence de médiateurs toxiques libérés par les cellules, l'absence de prolifération des macrophages activés, le fait que le rapport effecteur sur cellules cibles pour la cytotoxicité est plus bas que dans les autres traitements, et le fait qu'il ne nécessite pas l'injection simultanée de cytokine telle l'IL2 dont les effets secondaires sont néfastes. De plus, il permet de n'infecter qu'une population définie et controlée de cellules, il permet de choisir la multiplicité d'infection (nombre de particules virales par cellules), il permet d'atteindre de façon irréversible les tissus à partir de la circulation sanguine, et il permet de mettre à profit le rôle central des macrophages dans l'organisme aussi bien par leur activité anti-tumorale ou anti-infectieuse que dans leur activité de stimulation ou de régulation du système immunitaire comme expliqué plus haut. En outre, et compte-tenu de la durée de vie importante des macrophages, une répétition de traitements handicapante pour le malade pourrait être évitée.

La présente invention offre donc de nouvelles possibilités de traitement plus efficaces, moins astreignantes pour le malade, moins coûteuses, et plus reproductibles.

La présente invention sera davantage détaillée dans la description qui suit des possibilités de transformation des cellules de la lignée monocytes-macrophages et de l'expression du gène thérapeutique aussi bien au niveau de cellules en culture que de cellules réinjectées in vivo dans des tumeurs.

Les exemples donnés ne sont pas limitatifs mais sont une illustration de la faisabilité de la méthode et de la conservation de l'efficacité du gène et de la protéine exprimée par le gène transféré dans le macrophage aussi bien in vitro, que ex vivo, que in vivo.

### Légende des figures

Figure 1. Cette figure représente des coupes de macrophages dérivés des monocytes infectés par un adénovirus recombinant défectif portant le gène Lac-Z de E. coli (Ad RSV β gal), 48 heures après l'infection (Figure la), 4 jours après l'infection (Figure 1b), et 16 jours après l'infection (Figure 1c). Figure 1d : témoin cellules non infectées.

Figure 2. Détection de l'activité β galactosidase exprimée dans les monocytes-macrophages transformés par l'adénovirus Ad RSV β gal, après injection intratumorale dans des souris nude.

Les souris présentent des tumeurs induites par l'injection sous cutanée de cellules tumorales de mesotheliome humain, et sont traitées par injection intratumorale de monocytes-macrophages humains infectés par Ad RVS β gal. Trois jours après le traitement, les tumeurs sont extraites, fixées, et la présence de l'activité β galactosidase est révélée.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

Pour la production d'adénovirus, la lignée de rein embryonnaire humain 293 a été utilisée (Graham et al., J. Gen. Virol. 36 (1977) 59). Cette lignée contient notamment, intégrée dans son génome, la partie gauche du génome de l'adénovirus humain Ad5 (12 %).

### Exemples

### Exemple 1 : Préparation des monocytes et des macrophages

La technique de préparation des macrophages utilisée a été déjà décrite dans la littérature (Lopez et al., 1993, précitée). Brièvement les cellules mononuclées (MNC) ont été séparées des erythrocytes et granulocytes sur un gradient Ficoll-Hypaque (d=1,077) en utilisant le processeur de sang Cobe 2991, puis lavées trois fois avec un tampon phosphate. Une partie des MNC a été utilisée pour préparer des monocytes par élutriation dans les conditions données ci-dessous. L'autre partie des MNC a été mise en culture (5.10⁶ par ml) dans un milieu IMDM (Gibco, France) contenant 3.10⁻⁵ M de 2-mercaptoéthanol, 1% d'acides aminés non essentiels, 2mM de L-glutamine, 2mM pyruvate de sodium, 100 UI par ml de pénicilline, 100 µg par ml de streptomycine et 2% de sérum AB, dans des sacs en téflon de 600 ml puis incubée à 37°C dans une atmosphère humide à 5 % de C02 pendant 7 jours.

Les cellules ont ensuite été récupérées et les monocytes-macrophages purifiés à 95 % par élutriation en utilisant une centrifugeuse Beckman J6ME équipée avec un rotor J5.0 et une chambre d'élutriation de 40 ml.

### Exemple 2 : Infection des monocytes et des macrophages avec un vecteur adénoviral exprimant le gène de la β galactosidase (Ad. RSV βgal).

2.1. Préparation du vecteur : Le vecteur adénoviral utilisé dans cet exemple est le vecteur Ad.RSV.βgal. Ce vecteur est dépourvu des séquences nécessaires à sa réplication, mais comporte néanmoins les séquences nécessaires pour pénétrer dans les cellules infectables par celui-ci ainsi que l'ensemble des séquences essentielles nécessaires à l'encapsidation de cet adénovirus. Il porte également, sous le controle du promoteur RSV, le gène de la β galactosidase de E.coli. La construction de l'adénovirus recombinant défectif Ad.RSVβgal a été décrite dans la littérature (Stratford-Perricaudet et al., J. Clin. Invest. 90 (1992) 626). Brièvement, l'adénovirus Ad.RSVβGal est un adénovirus recombinant défectif (délété des régions E1 et E3 ) obtenu par recombinaison homologue in vivo entre l'adénovirus mutant Ad-d1324 (Thimmappaya et al., Cell 31 (1982) 543) et le plasmide pAd.RSVβGal (Akli et al. 1993).
Le plasmide pAd.RSVβGal contient, dans l'orientation 5'->3',
- le fragment PvuII correspondant à l'extrémité gauche de l'adénovirus Ad5 comprenant : la séquence ITR, l'origine de réplication, les signaux d'encapsidation et l'amplificateur E1A;
- le gène codant pour la β-galactosidase sous le contrôle du promoteur RSV (du virus du sarcome de Rous),
- un second fragment du génome de l'adénovirus Ad5, qui permet la recombinaison homologue entre le plasmide pAd.RSVβGal et l'adénovirus d1324.

Après linéarisation par l'enzyme ClaI, le plasmide pAd.RSVβGal et l'adénovirus d1324 sont co-transfectés dans la lignée 293 en présence de phosphate de calcium pour permettre la recombinaison homologue. Les adénovirus recombinants ainsi générés sont sélectionnés par purification sur plaque. Après isolement, l'ADN de l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.

Les particules virales sont généralement purifiées par centrifugation sur gradient de chlorure de césium selon les techniques connues (voir notamment Graham et al., Virology 52 (1973) 456).

Cet adénovirus a été utilisé pour démontrer la possibilité de transformer des monocytes-macrophages et pour montrer l'efficacité du transfert de gène dans ces cellules.

2.2. Infection des monocytes et macrophages : Des monocytes frais ou des macrophages ont été incubés pendant la nuit en présence de 80 à 100 PFU (Plaque forming unit) par cellule de virus purifé (Ad. RSV Bgal) dans un milieu RPMI complet (1.10⁶ cellules par ml). Les cellules ont été lavées pour éliminer les particules virales libres puis réincubées dans du milieu complet RPMI frais dans des sacs en téflon ou dans des plaques de culture de 12 puits. A différentes périodes, des aliquotes de cellules ont été lavés, fixés et testés pour la présence d'activité β galactosidase.

L'activité β galactosidase a été testée dans les cellules après infection par l'adénovirus recombinant en utilisant des méthodes histochimiques comme décrit dans (Stratford-Perricaudet et al., Hum. Gene Ther. 1 (1990) 241). Brièvement, les cellules sont incubées en présence de X-gal pour révéler l'activité β-gal qui est alors visualisée par apparition d'une coloration bleue dans le noyau des cellules contenant le gène β-gal, et contre-colorées avec de l'hémotoxyline et de l'éosine.

### Exemple 3 : Mise en évidence du transfert de gène dans les monocytes humains ou les macrophages dérivés de monocytes humains transformés par le vecteur adénoviral recombinant portant le gène de la β galactosidase.

3.1. Expression de l'activité B galactosidase dans les monocytes-macrophages en culture cellulaire.

L'efficacité du transfert de gène avec les vecteurs adénoviraux a été testée dans les monocytes purifiés et dans les macrophages dérivés de ces mêmes cellules monocytaires. Les cellules mononucléaires sanguines obtenues par cytaphérèse ont été séparées sur gradient de Ficoll comme décrit dans l'exemple 1. Une partie de la suspension de MNC a été soumise à élutriation et les monocytes ainsi obtenus ont été utilisés dans les essais d'infection (exemple 2). Une deuxième partie de la suspension de MNC a été cultivée pendant 6 à 7 jours permettant la différenciation des monocytes en macrophages comme décrit dans l'exemple 1. Après cette période de culture les macrophages ont également été purifiés par élutriation. Les macrophages ainsi purifiés sont deux fois plus gros que les monocytes, et expriment à leur surface l'antigène spécifique de la différenciation Max 1. L'expression du CD 14 de HLA-DR et de CD 64 est également augmentée de façon significative si on compare aux monocytes de départ. Une partie des macrophages obtenus à partir des monocytes en culture a ensuite été activée, par addition de 250 UI/:ml d'interféron gamma recombinant environ 18 heures avant la fin de la culture, selon le protocole décrit dans Lopez et al. (J. Immunotherapy 11 (1992) 209-217). Cette activation permet d'obtenir des macrophages activés susceptibles d'être actifs en immunothérapie anti-infectieuse.

Les monocytes purifiés ou les macrophages purifiés, soit après 6 jours de culture (c'est-à-dire avant activation par l'interféron gamma), soit après 7 jours de culture (après activation par l'interféron gamma), ont été infectés avec 80 à 100 pfu (pour plaque-forming units) par cellule de l'adénovirus recombinant Ad. RSV βgal.

Après infection pendant la nuit, le milieu est enlevé, les cellules lavées et réincubées dans un milieu complet frais, soit dans des sacs en téflon, soit dans des plaques douze puits. Les cellules ont ensuites été fixées et testées pour l'activité B galactosidase à différents intervalles de temps après l'infection. L'expression de la β galactosidase dans le noyau des cellules indique l'efficacité du transfert du gène et de son expression.

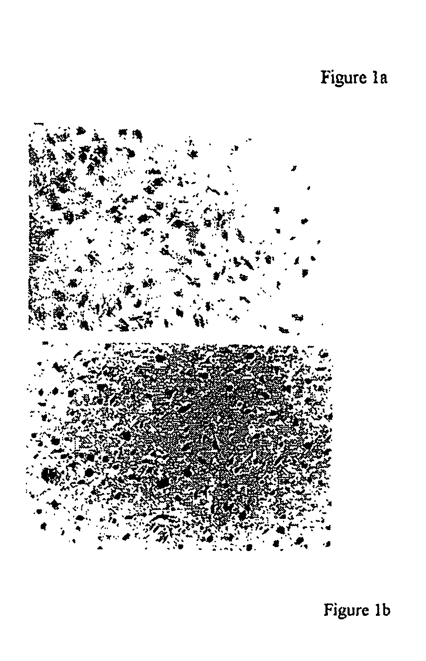

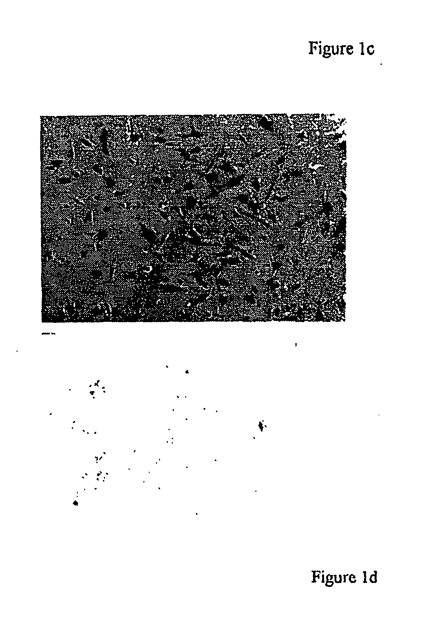

Les résultats obtenus sont présentés sur la figure 1. Ils montrent la détection de l'activité β galactosidase à différents temps après l'infection des macrophages dérivés de monocytes traités à l'interféron. Des résultats similaires sont obtenus avec des cellules non activées par l'interféron gamma. Selon la préparation, 40 à 80% des macrophages expriment l'activité β galactosidase dans leur noyau entre 2 et 4 jours après l'infection. L'expression de la β galactosidase continue jusqu'à 3 semaines après l'infection (figure 1C). Il est entendu que, en augmentant la multiplicité d'infection, le pourcentage de cellules exprimant le gène recombinant peut être augmenté. Il est ainsi possible d'obtenir des compositions cellulaires dans lesquelles 95 % ou plus des cellules sont transformées.

3.2. Expression de la β galactosidase après injection intratumorale des monocytes macrophages infectés par l'adénovirus recombinant dans des souris nude.

Dans l'optique de tester si les monocytes-macrophages infectés peuvent exprimer un gène exogène dans des tumeurs in vivo, les monocytes-macrophages infectés par l'adénovirus recombinant ont été injectés dans des tumeurs induites dans des souris nude.

Les souris nude ont tout d'abord été soumise à une injection sous-cutanée de 2.10⁶ cellules tumorales de mesotheliome humain (cellules HIB). Les tumeurs (d'un diamètre de 4 mm environ) induites par cette injection ont ensuite été traitées par injection intratumorale de monocytes-macrophages humains infectés par Ad.RVS βgal obtenus dans l'exemple 3.1. Pour l'injection, les monocytes-macrophages humains infectés ont été mis en suspension dans du tampon phosphate. Les doses injectées étaient de 2.10⁶ cellules par souris. Trois jours après le traitement, les tumeurs ont été extraites, fixées, et la présence de l'activité β galactosidase est révélée.

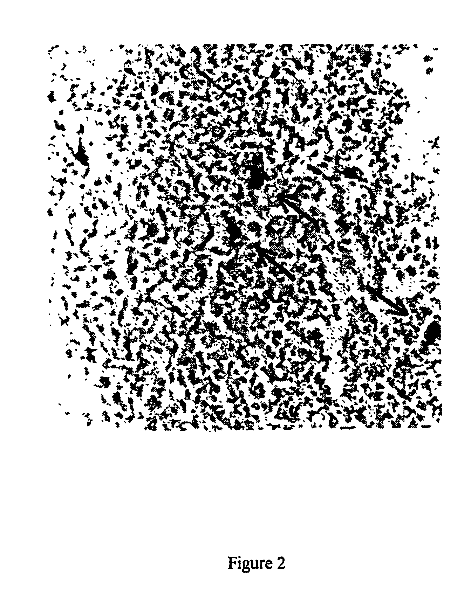

Les résultats obtenus sont présentés sur la figure 2. Ils montrent clairement que l'activité β galactosidase est trouvée dans la tumeur 3 jours après le traitement, et que les monocytes-macrophages administrés sont donc capables de coloniser de manière importante la tumeur et d'y exprimer un gène recombinant.

### Exemple 4 : Transfert d'un gène TNFα muté dans les macrophages dérivés de monocytes sanguins humains.

Le TNFα ou cachetine est une cytokine relarguée essentiellement à partir de macrophages en réponse à une destruction tissulaire, une endotoxine bactérienne, une affection virale et à d'autres cytokines. Le TNF est produit sous forme d'une pro-hormone de 233 acides amines (26kDa). La protéine mature comporte 157 acides amines (17kDa) et résulte de l'élimination d'une séquence N-terminale de 76 acides aminés. Les monocytes-macrophages activés synthétisent du TNFα membranaire et ont une action cytotoxique sur leur cible par contact cellule-cellule (effet du TNFα membranaire de 26kDa), ainsi que par libération locale de TNFα soluble (17kDa). Ceci est différent de ce qui se produit lors d'un choc septique, lequel choc résulterait partiellement d'une activation aigue ou chronique des monocytes entraînant la libération de la forme sécrétée de TNF (Ferez et al., Cell 63 (1990) 251). Depuis que le TNFα a été cloné et exprimé dans E.coli (Pennica et al. 1984), de grandes quantités sont disponibles pour des essais cliniques. Cependant l'utilisation thérapeutique du TNFα chez l'homme est limitée par sa toxicité importante et les effets secondaires qu'il génère (cachexie, fièvre, maux de tête, fatigue, hypertension). Dans le cancer colorectal, un essai clinique récent a montré qu'une toxicité importante apparaît à des doses insuffisantes pour obtenir une activité anti-tumorale (Kemeny et al., 1990).

La présente invention permet d'augmenter le pouvoir anti-tumoral des macrophages sans générer de toxicité liée au TNFα soluble. La présente invention permet en effet de générer des macrophages transfectés par la forme membranaire du TNFα. Par ailleurs, les macrophages de l'invention ainsi obtenus présentent un autre avantage important : en effet, alors que le TNFα soluble (secrété) a une demi-vie plasmatique courte d'environ 20 minutes, les macrophages de l'invention peuvent rester en périphérie de la tumeur pendant plusieurs jours, ce qui laisse espérer une activité anti-tumorale plus durable, résultant du contact macrophage portant le TNF-cellule tumorale.

Des macrophages ainsi activés ont été obtenus par infection de macrophages dérivés de monocytes ou de monocytes par des adénovirus recombinants tels que décrits dans l'exemple 2, mais dont le gène thérapeutique est celui d'un TNFα muté.

Plus précisément, le gène muté a été obtenu de la façon suivante. La forme membranaire du TNFα possède deux sites de clivage, l'un situé entre les acides aminés -1 et +1 (la position + 1 représente le début de la protéine mature), l'autre situé dans la région +13 (Perez et al., 1990, précitée). La délétion des acides aminés 1 à 12 empêche le clivage sous la forme 26kDa tout en maintenant la cytotoxicité aux zones de contact cellules-cellules. Les mutations du TNFα humain ont été réalisées par mutagénèse dirigée; elles correspondent aux mutants Δ+1, Δ1-12 décrites par C. Perez et al., 1990, précitée. L'efficacité des constructions de TNF muté est évaluée par expression transitoire dans des cellules eucaryotes (cos) en recherchant les formes membranaires, intracellulaires et sécrétées du TNF en utilisant des anticorps anti TNF et en mesurant son activité biologique. Un adénovirus recombinant comprenant le gène muté préparé ci-dessus est ensuite construit; et l'activité biologique du TNF synthétisé est controlée : (a) vis-à-vis de la lignée L 929 par la technique des zones de cytolyse en agar, laquelle technique révèle la lyse dépendant du contact cellules-cellules, et (b) par l'inhibition d'incorporation de thymidine tritiée dans les cellules en culture U 937 (cellules histocytaires d'un lymphome humain), K 562 (cellules provenant d'une leucémie myelogénique chronique) et LS 174 T (adénocarcinome colique) ainsi que vis à vis de cellules blastiques prélevées chez des patients leucémiques. L'activité anti-tumorale in vivo est ensuite testée dans un modèle animal, et consiste à implanter des tumeurs humaines à des souris nude puis à traiter ces souris avec des injections sytémiques de macrophages transfectés par le gène TNFα modifié et ce comme indiqué ci-dessus dans l'exemple 3. L'effet anti-tumoral est évalué par rapport à des souris témoins traitées par un placebo ou par des macrophages non transfectés.

### Exemple 5 : Macrophages dérivés de monocytes ou monocytes transformés par l'adénovirus recombinant contenant le gène de l'interféron gamma et conférant aux macrophages une activité anti-tumorale accrue.

Le gène de l'interféron gamma a été introduit dans le vecteur adénoviral décrit ci-dessus et l'adénovirus recombinant a été utilisé pour transformer des cellules de monocytes ou de macrophages dérivés de monocytes.

Les macrophages obtenus après différenciation dans le milieu RPMI comme décrit ci-dessus dans l'exemple 1 expriment l'interféron gamma de façon constitutive, il n'est donc plus nécessaire de traiter pendant 18 à 24 heures les macrophages pour obtenir l'effet cytotoxique et anti-tumoral requis. Les macrophages exprimant de façon constitutive l'interféron gamma peuvent être ainsi utilisés en immunothérapie anti-cancéreuse adoptive soit par injection systémique soit par injection directe dans les tumeurs.

## Revendications

1. Composition cellulaire comprenant des cellules de la lignée monocyte-macrophage différenciées contenant un acide nucléique recombinant, porté par un vecteur adénoviral comprenant, sous le contrôle d'éléments de régulation permettant son expression, un ou plusieurs gènes thérapeutiques.

2. Composition cellulaire selon la revendication 1 **caractérisée en ce que** le ou les gènes thérapeutiques codent pour tout ou partie d'une protéine capable de conférer aux cellules des propriétés anti-infectieuses, anti-cancéreuses ou immunostimulantes nouvelles ou accrues.

3. Composition cellulaire selon les revendications 1 ou 2 **caractérisée en ce que** le gène est choisi parmi les gènes codant pour les interférons, les facteurs de nécrose des tumeurs, les interleukines, les facteurs de stimulation des colonies (G-CSF, M-CSF, GM-CSF, etc), le gène MDR, et un gène codant pour un antigène d'une particule infectieuse ou spécifique d'une tumeur.

4. Composition cellulaire selon l'une des revendications 1 à 3 **caractérisée en ce que** le vecteur adénoviral est un adénovirus recombinant défectif.

5. Cellule précurseur de monocyte-macrophage contenant un adénovirus recombinant défectif comprenant, sous le contrôle d'éléments de régulation permettant son expression, un ou plusieurs gènes thérapeutiques.

6. Cellule précurseur selon la revendication 5, **caractérisée en ce qu'**elle est choisie parmi les cellules souches et progéniteurs du système hématopoïétique.

7. Composition pharmaceutique comprenant, à titre de principe actif, une ou plusieurs compositions cellulaires selon l'une des revendications 1 à 6.

8. Composition pharmaceutique selon la revendication 7 comprenant des cellules de la lignée monocyte-macrophage contenant un acide nucléique recombinant codant pour tout ou partie d'une protéine conférant au macrophage la propriété d'éliminer tout ou partie de cellules tumorales d'un organisme.

9. Composition pharmaceutique selon la revendication 8, **caractérisée en ce que** le gène est choisi parmi le gène de l'interféron- γ et le gène du facteur de nécrose des tumeurs alpha.

10. Composition pharmaceutique selon la revendication 7, comprenant des cellules de la lignée monocyte-macrophage contenant un adénovirus recombinant défectif portant le gène de l'INF-γ.

11. Composition pharmaceutique selon la revendication 7, comprenant des cellules de la lignée monocyte-macrophage contenant un adénovirus recombinant défectif portant le gène TNF-α.

12. Composition pharmaceutique selon la revendication 7, **caractérisée en ce que** l'adénovirus recombinant défectif porte un gène codant pour une forme membranaire du TNF-α.

13. Composition pharmaceutique selon la revendication 7, comprenant des cellules de la lignée monocyte-macrophage contenant un acide nucléique recombinant codant pour tout ou partie d'une protéine conférant au macrophage la propriété de prévenir ou de traiter les manifestations d'une infection par un agent infectieux tel un virus, un rétrovirus, un parasite ou une bactérie.

14. Composition pharmaceutique selon la revendication 13, **caractérisée en ce que** l'acide nucléique recombinant code pour un antigène de surface viral ou bactérien.

15. Composition pharmaceutique selon la revendication 7, comprenant des cellules de la lignée monocyte-macrophage contenant un adénovirus recombinant défectif exprimant un antigène de surface du virus HIV, tel que tout ou autre partie de la protéine GP 160.

16. Composition pharmaceutique selon l'une des revendications 7 à 15 **caractérisée en ce qu'**elle se présente sous forme injectable.

17. Composition pharmaceutique selon la revendication 16 en vue d'une injection intraveineuse ou intratumorale.

18. Composition pharmaceutique selon la revendication 16 formulée en vue d'une perfusion.

19. Composition pharmaceutique selon l'une des revendications 7 à 18 **caractérisée en ce qu'**elle comprend 10⁵ à 10⁹ cellules par dose.

20. Procédé de préparation de monocytes-macrophages selon l'une des revendications 1 à 6, **caractérisé en ce que** l'on effectue les étapes suivantes :
- la culture des cellules de monocytes-macrophages ou de leurs précurseurs prélevés et isolés à partir de sang ou de moelle osseuse,
- la transformation de ces cellules par un acide nucléique recombinant tel que défini ci-avant, et éventuellement,
- le conditionnement et/ou le stockage des cellules ainsi obtenues.

## Patentansprüche

1. Zellzusammensetzung, umfassend differenzierte Zellen der Monozyt-Makrophagen-Linie, die eine rekombinante Nucleinsäure enthält, welche von einem adenoviralen Vektor getragen wird, der unter der Kontrolle von ihre Expression erlaubenden Regulationselementen ein oder mehrere therapeutische Gene umfaßt.

2. Zellzusammensetzung gemäß Anspruch 1, **dadurch gekennzeichnet, dass** das oder die therapeutischen Gene für das gesamte Protein oder einen Teil eines Proteins kodieren, welches imstande ist, den Zellen neue oder verstärkte antiinfektiöse, antikanzeröse oder immunostimulierende Eigenschaften zu verleihen.

3. Zellzusammensetzung gemäß Anspruch 1 oder 2, **dadurch gekennzeichnet, dass** das Gen ausgewählt ist unter den Genen, die kodieren für die Interferone, die Nekrosefaktoren von Tumoren, die Interleukine, die Stimulationsfaktoren von Kolonien (G-CSF, M-CSF, GM-CSF usw.), dem MDR-Gen und einem Gen, das für ein Antigen eines infektiösen oder spezifischen Teilchens eines Tumors kodiert.

4. Zellzusammensetzung gemäß einem der Ansprüche 1 bis 3, **dadurch gekennzeichnet, dass** der adenovirale Vektor ein defektives rekombinantes Adenovirus ist.

5. Zellprecurser des Monozyt-Makrophagen, enthaltend ein defektives rekombinantes Adenovirus, welches unter der Kontrolle von ihre Expression erlaubenden Regulationselementen ein oder mehrere therapeutische Gene umfasst.

6. Zellvorläufer gemäß Anspruch 5, **dadurch gekennzeichnet, dass** er unter den Stammzellen und Progenituren des hämatopoietischen Systems ausgewählt ist.

7. Pharmazeutische Zusammensetzung, umfassend als Wirkstoff eine oder mehrerer Zellzusammensetzungen gemäß einem der Ansprüche 1 bis 6.

8. Pharmazeutische Zusammensetzung gemäß Anspruch 7, umfassend Zellen der Monozyt-Makrophagen-Linie, die eine rekombinante Nucleinsäure enthält, welche für das gesamte Protein oder einen Teil eines Proteins kodiert, das dem Makrophagen die Eigenschaft verleiht, sämtliche oder einen Teil der Tumorzellen eines Organismus zu eliminieren.

9. Pharmazeutische Zusammensetzung gemäß Anspruch 8, **dadurch gekennzeichnet, dass** das Gen unter dem Gen von γ-Interferon und dem Gen des Nekrosefaktors von alpha-Tumoren ausgewählt ist.

10. Pharmazeutische Zusammensetzung gemäß Anspruch 7, umfassend Zellen der Monozyt-Makrophagen-Linie, die ein defektives rekombinantes Adenovirus enthält, welches das Gen von γ-INF trägt.

11. Pharmazeutische Zusammensetzung gemäß Anspruch 7, umfassend Zellen der Monozyt-Makrophagen-Linie, die ein defektives rekombinates Adenovirus enthält, das das α-TNF-Gen trägt.

12. Pharmazeutische Zusammensetzung gemäß Anspruch 7, **dadurch gekennzeichnet, dass** das defektive rekombinante Adenovirus ein Gen trägt, welches für eine membranäre Form von α-TNF kodiert.

13. Pharmazeutische Zusammensetzung gemäß Anspruch 7, umfassend Zellen der Monozyt-Makrophagen-Linie, die eine rekombinante Nucleinsäure enthält, welche für das gesamte oder einen Teil eines Proteins kodiert, das dem Makrophagen die Eigenschaft verleiht, Äußerungen einer Infektion durch ein infektiöses Mittel, wie ein Virus, ein Retrovirus, ein Parasit oder eine Bakterie, zu verhüten oder zu behandeln.

14. Pharmazeutische Zusammensetzung gemäß Anspruch 13, **dadurch gekennzeichnet, dass** die rekombinante Nucleinsäure für ein Antigen der viralen oder bakteriellen Oberfläche kodiert.

15. Pharmazeutische Zusammensetzung gemäß Anspruch 7, umfassend Zellen der Monozyt-Makrophagen-Linie, die ein defektives rekombinantes Adenovirus enthält, welches ein Antigen der Oberfläche des HIV-Virus, wie das gesamte oder einen anderen Teil des Proteins GP160, exprimiert.

16. Pharmazeutische Zusammensetzung gemäß einem der Ansprüche 7 bis 15, **dadurch gekennzeichnet, dass** sie in injizierbarer Form vorliegt.

17. Pharmazeutische Zusammensetzung gemäß Anspruch 16 zur intravenösen oder intratumoralen Injektion.

18. Pharmazeutische Zusammensetzung gemäß Anspruch 16, die im Hinblick auf eine Perfusion formuliert ist.

19. Pharmazeutische Zusammensetzung gemäß einem der Ansprüche 7 bis 18, **dadurch gekennzeichnet, dass** sie 10⁵ bis 10⁹ Zellen je Dosis umfasst.

20. Verfahren zur Herstellung von Monozyt-Makrophagen gemäß einem der Ansprüche 1 bis 6, **dadurch gekennzeichnet, dass** man die folgenden Stufen durchführt:
- die Kultur der Zellen von Monozyt-Makrophagen oder ihren Precursern, die aus dem Blut oder dem Knochenmark entnommen und isoliert worden sind,
- die Transformation dieser Zellen mit einer rekombinanten Nucleinsäure, wie vorstehend definiert, und gegebenenfalls
- die Konditionierung und/oder Lagerung der so erhaltenen Zellen.

## Claims

1. Cell composition comprising differentiated cells of the monocyte-macrophage line containing a recombinant nucleic acid, carried by an adenoviral vector comprising, under the control of regulatory elements permitting its expression, one or more therapeutic genes.

2. Cell composition according to Claim 1, **characterized in that** the therapeutic gene or genes code(s) for all or part of a protein capable of endowing the cells with novel or increased anti-infectious, anticancer or immunostimulatory properties.

3. Cell composition according to Claim 1 or 2, **characterized in that** the gene is chosen from the genes coding for interferons, tumour necrosis factors, interleukins and colony stimulating factors (G-CSF, M-CSF, GM-CSF, etc.), the MDR gene and a gene coding for an antigen of an infectious particle or specific to a tumour.

4. Cell composition according to one of Claims 1 to 3, **characterized in that** the viral vector is a defective recombinant adenovirus.

5. Precursor cell of monocytes-macrophages containing a defective recombinant adenovirus comprising, under the control of regulatory elements permitting its expression, one or more therapeutic genes.

6. Precursor cell according to Claim 5, **characterized in that** it is chosen from stem and progenitor cells of the haematopoietic system.

7. Pharmaceutical composition comprising as active principle one or more cell compositions according to one of Claims 1 to 6.

8. Pharmaceutical composition according to Claim 7, comprising cells of the monocyte-macrophage line containing a recombinant nucleic acid coding for all or part of a protein endowing the macrophage with the property of eliminating all or part of tumour cells from an organism.

9. Pharmaceutical composition according to Claim 8, **characterized in that** the gene is chosen from the interferon-γ gene and the tumour necrosis factor alpha gene.

10. Pharmaceutical composition according to Claim 7, comprising cells of the monocyte-macrophage line containing a defective recombinant adenovirus carrying the INF-γ gene.

11. Pharmaceutical composition according to Claim 7, comprising cells of the monocyte-macrophage line containing a defective recombinant adenovirus carrying the TNFα gene.

12. Pharmaceutical composition according to Claim 7, **characterized in that** the defective recombinant adenovirus carries a gene coding for a membrane form of TNFα.

13. Pharmaceutical composition according to Claim 7, comprising cells of the monocyte-macrophage line containing a recombinant nucleic acid coding for all or part of a protein endowing the macrophage with the property of preventing or treating manifestations of an infection by an infectious agent such as a virus, a retrovirus, a parasite or a bacterium.

14. Pharmaceutical composition according to Claim 13, **characterized in that** the recombinant nucleic acid codes for a viral or bacterial surface antigen.

15. Pharmaceutical composition according to Claim 7, comprising cells of the monocyte-macrophage line containing a defective recombinant adenovirus expressing a surface antigen of the HIV virus, such as all or part of the GP160 protein.

16. Pharmaceutical composition according to one of Claims 7 to 15, **characterized in that** it is in injectable form.

17. Pharmaceutical composition according to Claim 16, intended for intravenous or intratumoral injection.

18. Pharmaceutical composition according to Claim 16, formulated for the purpose of perfusion.

19. Pharmaceutical composition according to one of Claims 7 to 18, **characterized in that** it comprises 10⁵ to 10⁹ cells per dose.

20. Method of preparation of monocytes-macrophages according to one of Claims 1 to 6, **characterized in that** the following steps are performed:
- culturing of the monocyte-macrophage cells or their precursors withdrawn and isolated from blood or bone marrow,
- transformation of these cells with a recombinant nucleic acid as defined above, and, where appropriate,
- packaging and/or storage of the cells thereby obtained.
